# EUROPÄISCHE PATENTSCHRIFT

(11) **EP 0 989 405 B1**
(45) Veröffentlichungstag und Bekanntmachung des Hinweises auf die Patenterteilung: **09.07.2003**
(21) Anmeldenummer: 99118732.9
(22) Anmeldetag: 22.09.1999
(51) Int. Cl.: G01N 33/18, G01N 1/10

(54) **Vorrichtung und Verfahren zur Analyse von chemischen Substratgehalten in einer Flüssigkeit**
Device and method for analysing the contents of chemical substances in a liquid
Dispositif et procédé de détermination des teneurs des substances chimiques d'un liquide

(30) Priorität: 23.09.1998 DE 19843750
(43) Veröffentlichungstag der Anmeldung: 29.03.2000
(73) Patentinhaber: WTW Wissenschaftlich-Technische Werkstätten GmbH & Co. KG, 82362 Weilheim (DE)
(72) Erfinder: Schwab, Ulrich, 82166 Graefelfing (DE); Essel, Raimund, 82362 Weilheim (DE); Stäuble, Albert, 82383 Hohenpeissenberg (DE); Schuhmacher, Rudolf, 82380 Peissenberg (DE)
(74) Vertreter: Zipse + Habersack

(56) Entgegenhaltungen:
- WO-A-97/19026
- DE-C- 4 412 284
- US-A- 5 505 854
- US-A- 5 690 830
- US-A- 5 769 539
- PATENT ABSTRACTS OF JAPAN vol. 016, no. 009 (P-1296), 10. Januar 1992 (1992-01-10) & JP 03 229129 A (SUMITOMO JUKIKAI ENVIROTEC KK), 11. Oktober 1991 (1991-10-11)
- PATENT ABSTRACTS OF JAPAN vol. 012, no. 170 (P-705), 20. Mai 1988 (1988-05-20) & JP 62 282263 A (KAWASAKI STEEL CORP), 8. Dezember 1987 (1987-12-08)

## Beschreibung

Die Erfindung betrifft eine Vorrichtung gemäß dem Oberbegriff des Patentanspruchs 1 und ein Verfahren gemäß dem Oberbegriff des Patentanspruchs 8 zur Analyse von chemischen Substratgehalten in einer Flüssigkeit, insbesondere einer Klärlösung.

In herkömmlichen verfahrenstechnischen Prozessen, wie beispielsweise der Klärung von Abwässern durch Prozeßwasser-Aufbereitungsanlagen, ist es erforderlich, das Ergebnis der Aufbereitung zu überprüfen. Die Qualität der Aufbereitung hängt in erster Linie davon ab, wie hoch der Anteil an Phosphat, Nitrat und Ammonium-Stickstoff ist, der als Rückstand in der Lösung verbleibt. Man bedient sich hierzu eines Analyseverfahrens, bei dem das Prozeßwasser einer Vorrichtung zugeführt wird, die aus einer Filtereinheit und einer Analysevorrichtung besteht. Zunächst wird das Prozeßwasser der Filtereinheit, insbesondere einer Filtermembran, zugeführt. Diese Filtermembran erfüllt die Aufgabe, Feststoffpartikelchen des Prozeßwassers zurückzuhalten, so daß dem Analysegerät eine geeignete Form des Prozeßwassers zugeführt wird, um somit den Anteil an Phosphaten, Nitraten und Ammonium-Stickstoff analysieren zu können. Das Analysegerät ist ein hochempfindliches Meßgerät, angeschlossen an weitere elektronische Komponenten und sie befindet sich aus diesem Grunde vorteilhaft in einem Labor. Herkömmlicherweise befindet sich das Filtermedium im Prozeßwasseraufbereitungsbecken und ist über eine Leitung, durch die die zur Analyse vorgesehene Flüssigkeit geführt wird, mit dem Analysegerät verbunden. Ein Nachteil dieses bestehenden Verfahrensaufbaus liegt darin, daß diese Leitung zumeist sehr lang ist, da das Labor vom Prozeßwasser-Aufbereitungsbecken räumlich entfernt ist. Muß nun die zur Analyse vorgesehene Flüssigkeit über längere Strecken in einer Leitung gefördert werden, kann sich der Anteil an den zu untersuchenden Substraten, insbesondere an Phosphat und Ammonium-Stickstoff bzw. Nitrat ändern, da auch in dieser Zuleitung chemische Prozesse ablaufen, die zumeist diesen Gehalt ändern. Ein weiterer Nachteil des bestehenden Verfahrensaufbaus besteht darin, daß die besagte Zuleitung zum Analysegerät zumeist außerhalb eines Gebäudes liegt, wodurch eine Heizvorrichtung vorgesehen werden muß, damit die zu analysierende Flüssigkeit insbesondere im Winter nicht einfrieren kann. Die Anfälligkeit der Zuleitung einzufrieren ist auch deshalb so hoch, da die Durchlaufleistung der Filtermembran begrenzt ist und nur eine kleine Menge der zu analysierenden Flüssigkeit von der Filtermembran abgeschieden wird. Dadurch ist es erforderlich, Zuleitungen mit einem kleinen Leitungsquerschnitt zu verwenden, die dann allerdings auch leichter einfrieren.

Die Aufgabe der Erfindung liegt nun darin, die oben beschriebenen Nachteile zu eliminieren und ein Verfahren und eine Vorrichtung zu schaffen, die eine genaue und problemlose Analyse der chemischen Substratgehalte ermöglichen.

Die Aufgabe der Erfindung wird gelöst durch die Merkmale der nebengeordneten Ansprüche 1 und 8. Vorteilhafte Weiterbildungen der Erfindung sind Gegenstand der Unteransprüche.

Eine Vorrichtung gemäß dem Oberbegriff des Anspruchs 1 ist aus dem Dokument US-A-5767539 bekannt. Weiterhin wurde in dem Dokument US-A-5690830 schon vorgeschlagen, bei einer Filtrationsvorrichtung in einer Kläranlage die Reinigung des Filtermediums mittels Gasbeaufschlagung zu verbessern.

Grundgedanke der Erfindung ist es, eine Vorrichtung mit einem separaten in sich geschlossenen Filtrationsraum zu schaffen, der es ermöglicht, die Probenaufbereitung separat vom Klärbekken und in räumlicher Nähe zum Analysegerät durchzuführen. Hierdurch kann die besagte Zuleitung für das Filtrat zum Analysegerät so kurz wie möglich gehalten werden. Die Filtrationsvorrichtung umfaßt deshalb ein separates Filtrationsgehäuse, dem das Prozeßwasser, beispielsweise über eine Pumpe zugeführt wird, und eine in dem Filtrationsgehäuse angeordnete Gaszufuhreinrichtung, die dazu vorgesehen ist, das in die Flüssigkeit eingetauchte Filtermedium mit einem Gasstrom zu beaufschlagen, bzw. zu umströmen, der ein Zusetzen des Filtermediums mit Schmutzpartikeln verhindert oder zumindest verzögert. Die Zuleitung des Prozeßwassers zum Filtrationsgehäuse erfolgt mit einem - verglichen zur Filtrationszuleitung - sehr hohen Volumenstrom und über eine entsprechend stärker dimensionierte Leitung, wodurch ein Einfrieren im Winterbetrieb wirkungsvoll unterbunden werden kann. Die Filtrationsvorrichtung ist dabei in einen Kreislauf eingebettet, in dem überschüssiges Prozeßwasser wieder dem Prozeßwasser-Aufbereitungsbecken, insbesondere dem Klärbecken zurück geführt wird.

Die Zufuhr des Filtrats von der Filtrationsvorrichtung zum Analysegerät erfolgt jedoch entweder über eine äußerst kurze Zuleitung aufgrund der räumlich nahen Anordnung des Filtrationsgehäuses zum Analysegerät und/oder in einer temperierten Umgebung, z.B. im Betriebsgebäude. Somit erfolgt die Filtratzuleitung ungestört und unabhängig von jahreszeitlichen Einflüssen. Durch das Zusammenwirken der oben genannten Merkmale wird die Genauigkeit der Messung erhöht. Bei einer vorteilhaft verwendeten kurzen Filtratzuleitung zum Analysegerät werden chemische Prozesse in der Zuleitung , die die chemischen Substratgehalte der eigentlichen Klärung verfälschen könnten, vermieden.

Es ist prinzipiell möglich, einen Gasstrom auf das Filtermedium zu richten. In einer vorteilhaften Weiterbildung ist jedoch das Filtermedium, insbesondere eine flache Filtermembran, oberhalb der besagten Gaszufuhreinrichtung angeordnet, so daß die nach oben steigenden Gasblasen an der Oberfläche der Membran Turbulenzen erzeugen, womit sich ein Gleichgewicht zwischen Schmutzauftrag durch Filtration und Schmutzabtrag durch Turbulenzen einstellt. Durch diesen wie gerade beschriebenen Schmutzabtrag ist ein Prozeß gesteuert, der die Membranen kontinuierlich funktionsfähig hält oder zumindest das Zusetzen des Filtermediums verzögert.

Sowohl für die Zufuhr des Prozeßwassers in das Filtrationsgehäuse als auch für die Zufuhr des zu analysierenden Filtrats zum Analysegerät kann jeweils eine Pumpe vorgesehen sein.
Um Investitionskosten zu sparen, ist es möglich, die Gaszufuhreinrichtung in den Rahmen zu integrieren, an dem die Filtermembran befestigt ist. Das geschieht in der Weise, daß der untere Teil des Rahmens als Kanal zur Gaseinleitung ausgebildet und mit Austrittsöffnungen versehen ist, die das Austreten des Gases in das Prozeßwasser im Filtrationsgehäuse der Filtrationseinrichtung ermöglichen. Dieses Gas kann vorteilhaft Luft sein, da keine weiteren Anforderungen an das Gas gestellt sind, als besagte Turbulenzen an der Oberfläche der Membran hervorzurufen. Mit Vorteil kann ein Gebläse genutzt werden, das das Gas in das Filtrationsgehäuse der Filtrationseinrichtung einleitet.

In einer vorteilhaften Weiterbildung der Erfindung können mehrere Filtermedien in einem Filtrationsgehäuse angeordnet sein. Hierdurch läßt sich die wirksame Filteroberfläche und damit die erwünschte Filtratmenge erhöhen. Die Filtermedien können jedoch auch alternierend betrieben werden. Dann ist eine wirksamere Reinigungsleistung durch die Turbulenzeinwirkung des Gasstroms auf die Membran gegeben, die nicht zur Substratabscheidung betrieben wird. Zusätzlich ist damit auch die Möglichkeit geschaffen, die Membranen sowie die Ableitungen dieser nicht in Filterfunktion stehenden Membranen gegenspülen zu können, ohne den eigentlichen Betrieb der Filtration zu unterbrechen. Vorteilhafterweise ist eine Steuerung vorgesehen, die den alternierenden Betrieb der Filtermedien zwischen Filtration und Gegenspülung steuert.

Tritt der Fall ein, die Filtrationsvorrichtung zu Wartungs- bzw. Reparaturzwecken ausbauen zu müssen, ist vorzugsweise an der Zulaufleitung des Prozeßwassers eine Abzweigleitung mit Absperrvorrichtung vorgesehen, wodurch die Flüssigkeit problemlos zurückgeleitet werden kann, um die Filtrationsvorrichtung zu umgehen. Ein in das System integrierter Ablauf sorgt dafür überschüssiges Prozeßwasser zusammen mit den abgeschiedenen Feststoffpartikeln in das Klärbecken zurückzuführen. Es ist somit weiterhin gewährleistet, daß der Filtrationsvorrichtung ständig neues Prozeßwasser zu- bzw. abgeführt wird, und daß die zu analysierende Flüssigkeit nicht gealtert ist, bis sie das Analysegerät erreicht.
Um eine einfache und zuverlässige Steuerung der gesamten Apparatur zu gewährleisten, kann eine Steuerzentrale (Prozeßsteuerung) vorgesehen sein, von der aus jeder einzelne Prozeßschritt wie Gaszufuhr, Prozeßwasserzufuhr, Förderung der zu analysierenden Flüssigkeit, einschließlich der oben erwähnten Steuereinheit für den alternierenden Betrieb, ausgeführt werden kann. Dies kann auch durch eine Steuerung des Analysegerätes realisiert sein.

Die Funktionsfähigkeit des eben geschilderten Verfahrens ist nicht unbedingt von der Verwendung flacher Filtermembranen abhängig, es können hier auch herkömmliche Membrankapillare oder anders geformte Filtermedien verwendet werden.

Die Erfindung wird nachfolgend beispielsweise anhand einer Schemazeichnung beschrieben. In dieser zeigen:
- Figur 1 einen schematischen Aufbau einer Filtrationsvorrichtung,
- Figur 2 ein Detail aus Figur 1, zum Gasauftrag auf das Filtermedium

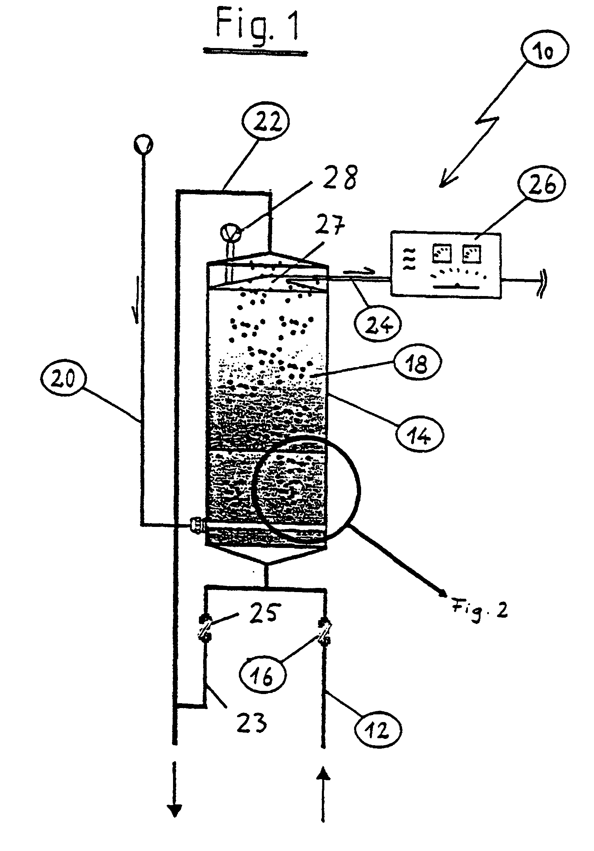

Figur 1 zeigt den schematischen Aufbau der Filtrationsvorrichtung 10. Die Zufuhr des Prozeßwassers erfolgt über die Zuleitung 12 in das Filtrationsgehäuse 14. Diese kann vorteilhaft über eine Absperrvorrichtung 16 (beispielsweise Kugelhahn) unterbrochen werden, damit ein Austausch oder eine Wartung des Filtrationsgehäuses problemlos möglich ist. Im Gehäuse befindet sich das Filtrationsmedium, hier in Form einer flach ausgebildeten Membran 18. Ein Gas, insbesondere Luft, wird dem Filtrationsgehäuse über eine Gasleitung 20 zugeführt. Ein Überlauf 22 sorgt für den Abtransport von abgeschiedenen mit den Gasblasen als auch mit dem nach oben gerichteten Flüssigkeitsstrom nach oben transportierten Partikeln des überschüssigen Prozeßwassers. Weiterhin kann das Filtrationsgehäuse 14 über einen in den Boden mündenden Ablauf 23 zu Wartungszwecken entleert werden. Der Ablauf ist im normalen Betrieb über das Ventil 25 gesperrt. Vom Filtratraum 27 im Filtrationsgehäuse 14 führt eine Filtrationsleitung 24 zum Analysegerät 26. Mit dem Filtratraum 27 ist weiterhin eine Gegenspülvorrichtung 28 verbunden, die den Filtratraum unter Druck mit Gas und/oder Flüssigkeit beaufschlagen kann, um die Filtermembran 18 zu reinigen. Vorzugsweise bildet die Leitung 20 den unteren Teil des Rahmens bildet, in dem die Membran gehalten ist, wobei diesem Rahmenteil Austrittsöffnungen 29 für das Gas vorgesehen sind.

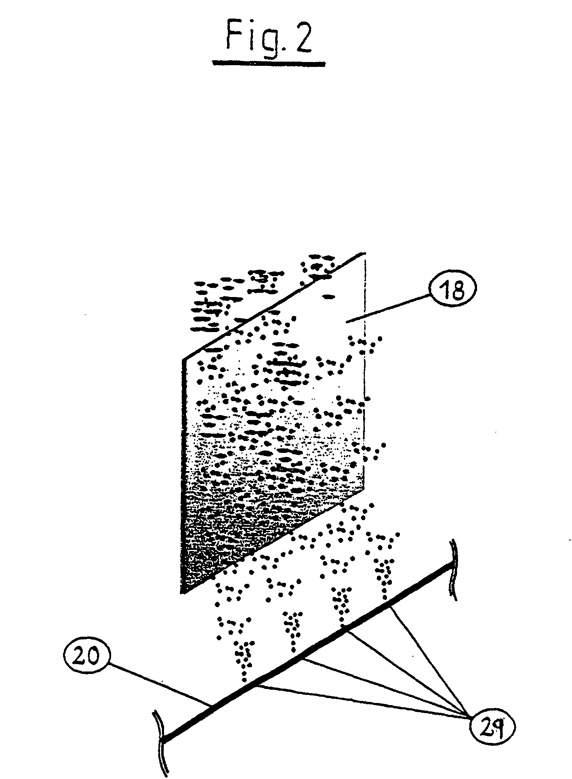

Figur 2 zeigt die Gasbeaufschlagung des Filtermediums, das hier als flache Filtermembran 18 ausgebildet ist. Dabei strömt das Gas aus Austrittsöffnungen 29 der Gaszufuhrleitung 20 nach oben und strömt über die Oberfläche der Filtermembran, wodurch mechanisch Schmutz von der Oberfläche gelöst und nach oben gespült wird.

## Patentansprüche

1. Vorrichtung zur Analyse von chemischen Substratgehalten in einer Flüssigkeit, insbesondere einer Klärlösung oder eines Prozeßwassers, die ein Analysegerät (26) und eine Filtrationsvorrichtung (10) zum Filtern der Flüssigkeit umfaßt, deren Filtrat dem Analysegerät (26) zuleitbar ist,
wobei die Filtrationsvorrichtung (10) ein separates Filtrationsgehäuse (14) umfaßt, in den ein Filtermedium (18) angeordnet ist
**gekennzeichnet durch** eine in dem Filtrationsgehäuse (14) angeordnete Gaszufuhreinrichtung, wodurch die Filtrationsvorrichtung (10) mittels Gasbeaufschlagung gereinigt wird.

2. Vorrichtung gemäß dem Anspruch 1,
**dadurch gekennzeichnet, daß** die Filtrationsvorrichtung (10) benachbart zum Analysegerät (26) angeordnet ist.

3. Vorrichtung gemäß dem Anspruch 1 und 2,
**dadurch gekennzeichnet, daß** das Filtermedium als Membran (18) ausgebildet ist, die auf einem Rahmen befestigt ist, und daß die Membran (18) über der im unteren Bereich des Filtrationsgehäuses (14) angebrachten Gaszufuhreinrichtung (20) angeordnet ist.

4. Vorrichtung gemäß einem der vorangehenden Ansprüche,
**dadurch gekennzeichnet, daß** das Filtermedium in Form einer flächigen Membran (18) oder in Form von Membrankapillaren ausgebildet ist.

5. Vorrichtung gemäß einem der vorangehenden Ansprüche,
**dadurch gekennzeichnet, daß** mindestens zwei, vorzugsweise mehr Filtermedien in dem Filtrationsgehäuse (14) angeordnet sind.

6. Vorrichtung gemäß einem der vorangehenden Ansprüche,
**dadurch gekennzeichnet, daß** eine Druckerzeugungsvorrichtung, insbesondere eine Pumpe, als Gegenspülvorrichtung (28) mit dem Filtratraum (27) der Filtrationsvorrichtung (10) verbunden ist.

7. Vorrichtung gemäß dem Anspruch 5 und 6,
**dadurch gekennzeichnet, daß** eine Steuerung vorgesehen ist, die gleichzeitig bei einigen der Filtermembranen einen Filtrationsbetrieb und bei anderen Filtermembranen einen Gegenspülbetrieb zur Reinigung verschmutzter Filtermedien steuert.

8. Verfahren zur Analyse von chemischen Substratgehalten in einer Flüssigkeit, insbesondere einer Klärlösung unter Anwendung einer Vorrichtung gemäß dem Anspruch 1.

9. Verfahren gemäß dem Anspruch 8,
**dadurch gekennzeichnet, daß** mehrere Filtermedien verwendet werden.

10. Verfahren gemäß dem Anspruch 8 und 9,
**dadurch gekennzeichnet, daß** die Filtermedien mit einer Gegenspülvorrichtung (28) gegengespült werden und Rückstände so entfernt werden.

11. Verfahren gemäß den Ansprüchen 8 bis 10,
**dadurch gekennzeichnet, daß** von den mehreren Filtermedien gleichzeitig einige im Filtratbetrieb und andere im Gegenspülbetrieb betrieben werden.

## Claims

1. An apparatus for analyzing the amount of chemical substrates in a liquid, more particularly in a clarified solution or a process water, comprising an analyzer (26) and a filtration device (10) for filtering the liquid and the filtrate of which may be fed to said analyzer (26), said filtration device (10) comprising a separate filtration housing (14) in which a filter medium (18) is accommodated, **characterized by** a gas supply means (20) fitted in said filtration housing (14), resulting in said filtration device (10) being cleaned by being charged with gas.

2. The apparatus as set forth in claim 1, **characterized in that** said filtration device (10) is arranged in the vicinity of said analyzer (26).

3. The apparatus as set forth in claim 1 and 2, **characterized in that** said filter medium (18) is configured as a membrane (18) secured to a frame and said membrane (18) being arranged above said gas supply means (20) fitted in the lower part of said filtration housing (14),

4. The apparatus as set forth in any of the preceding claims, **characterized in that** said filter medium (18) is configured in the form of a sheet membrane (18) or in the form of membrane capillaries.

5. The apparatus as set forth in any of the preceding claims, **characterized in that** at least two, preferably more, filter media are arranged in said filtration housing (14).

6. The apparatus as set forth in any of the preceding claims, **characterized in that** a pressurizing means, more particularly a pump is connected as a backwasher (28) to the filtrate space (27) of said filtration device (10).

7. The apparatus as set forth in claim 5 and 6, **characterized in that** a controller is provided simultaneously controlling a filtration mode and a backwash mode when said filter media are soiled.

8. A method for analyzing the amount of chemical substrates in a liquid, more particularly in a clarified solution with application of an apparatus as set forth in claim 1.

9. The method as set forth in claim 8, **characterized in that** several filter media are made use of.

10. The method as set forth in claim 8 and 9, **characterized in that** said filter media are backwashed by a backwasher (28) to thus remove residues.

11. The method as set forth in any of the claims 8 to 10 **characterized in that** several filter media are operated at the same time in the filtrate mode and backwash mode.

## Revendications

1. Dispositif de détermination des teneurs des substances chimiques dans un liquide, notamment d'un liquide d'épuration ou d'eau de procédé , disposant d'un appareil d'analyse (26) et d'un dispositif de filtration (10) pour filtrer le liquide ; dont le filtrat est amené vers l'appareil d'analyse (26) ; le dispositif de filtration (10) comportant un boîtier de filtration (14) dans lequel est disposé un moyen filtrant (18), **caractérisé par** un dispositif d'approvisionnement en gaz disposé dans le boîtier de filtration (14) ; le dispositif de filtration (10) étant nettoyé au moyen d'une exposition au gaz.

2. Dispositif selon la revendication 1, **caractérisé en ce que** le dispositif de filtration (10) est disposé près de l'appareil d'analyse (26).

3. Dispositif selon la revendication 1 ou 2, **caractérisé en ce que** le moyen filtrant a la forme d'une membrane (18) qui est fixée au cadre et **caractérisé en ce que** la membrane (18) est disposée au dessus du dispositif d'approvisionnement en gaz (20) installé dans la zone inférieure du boîtier de filtration (14).

4. Dispositif selon une des revendications précédentes, **caractérisé en ce que** le moyen filtrant a la forme d'une membrane plane (18) ou la forme de membranes capillaires .

5. Dispositif selon une des revendications précédentes, **caractérisé en ce qu'**au moins deux, de préférence, plusieurs moyens filtrants sont disposés dans le boîtier de filtration (14).

6. Dispositif selon une des revendications précédentes, **caractérisé en ce qu'**un dispositif de production de pression, notamment une pompe, est relié en tant que dispositif de rinçage en sens inverse (28) avec la chambre de filtrat (27) du dispositif de filtration (10).

7. Dispositif selon la revendication 6 et 7, **caractérisé en ce qu'**il est prévu une commande qui permet de commander en même temps une filtration pour certaines des membranes filtrantes et pour d'autres membranes filtrantes, un fonctionnement de rinçage en sens inverse afin de nettoyer des moyens filtrants salis.

8. Procédé pour analyser des teneurs de substances chimiques dans un liquide, notamment d'un liquide d'épuration en utilisant un dispositif conforme à la revendication 1.

9. Procédé selon la revendication 8, **caractérisé en ce que** plusieurs moyens filtrants sont utilisés.

10. Procédé selon la revendication 8 ou 9, **caractérisé en ce que** les moyens filtrants sont rincés en sens inverse avec un dispositif de rinçage en sens inverse (28) et que les résidus sont ainsi éliminés.

11. Procédé selon les revendications 8 à 10, **caractérisé en ce que** parmi les différents moyens filtrants , on fait fonctionner certains en même temps en fonctionnement de filtrat et d'autres en fonctionnement de rinçage en sens inverse.
